(19) Europäisches Patentamt
European Patent Office
Office européen des brevets

(11) **EP 3 590 563 B1**

(12) **EUROPEAN PATENT SPECIFICATION**

(45) Date of publication and mention
of the grant of the patent:
**23.09.2020 Bulletin 2020/39**

(51) Int Cl.:
**A61M 1/36** (2006.01)     **A61M 1/34** (2006.01)
**A61M 1/16** (2006.01)

(21) Application number: **18761762.6**

(22) Date of filing: **22.02.2018**

(86) International application number:
**PCT/JP2018/006467**

(87) International publication number:
**WO 2018/159452 (07.09.2018 Gazette 2018/36)**

(54) **BLOOD PURIFICATION DEVICE**

BLUTREINIGUNGSVORRICHTUNG

DISPOSITIF DE PURIFICATION DE SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.03.2017 JP 2017039750**

(43) Date of publication of application:
**08.01.2020 Bulletin 2020/02**

(73) Proprietor: **Asahi Kasei Medical Co., Ltd.
Tokyo 1000006 (JP)**

(72) Inventors:
• **UMIMOTO, Takashi
60528 Frankfurt am Main (DE)**
• **FONSECA SAN MIGUEL Fernando
01004 Vitoria-Gasteiz, Alava (ES)**
• **MAYNAR MOLINER Javier
01004 Vitoria-Gasteiz, Alava (ES)**

• **BARRASA GONZALEZ Helena
01004 Vitoria-Gasteiz, Alava (ES)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
WO-A1-2016/172238    JP-A- 2002 248 165
JP-A- 2009 527 343    JP-A- 2014 511 211
JP-A- 2016 087 438    US-A- 4 500 309
US-A1- 2002 107 469    US-A1- 2008 015 487
US-A1- 2011 168 614    US-A1- 2011 208 105
US-A1- 2014 353 251    US-B2- 9 039 647

EP 3 590 563 B1

## Description

## Technical Field

The present invention relates to a blood purification apparatus.

## Background Art

**A** blood purification process that is performed in a dialysis treatment, a plasma exchange treatment, and the like is generally performed using a blood purification apparatus. Blood purification apparatuses include a blood purifier and a blood circuit that sends blood collected from a patient to the blood purifier and returns blood purified in the blood purifier to the patient.

In order to prevent a patient's blood from coagulating in the blood circuit, an anticoagulant has been supplied to the blood circuit upstream from the blood purifier in the related art.

Heparin has been used as an anticoagulant in the related art. However, in recent years, use of citric acid which imposes less of a burden on a blood purification membrane of the blood purifier and can be used for patients with bleeding disorders has been proposed.

However, when citric acid is used as an anticoagulant, since an amount of ionized calcium in blood is reduced due to the action of citric acid, an amount of Ci-Ca conjugates (a type of calcium complex) increases, and a large amount thereof is removed by the blood purification membrane in the blood purifier, a calcium concentration in blood that is returned to the patient after the blood purifier has passed is significantly lowered. Therefore, a device configured to supply calcium to the blood circuit downstream from the blood purifier is provided in the blood purification apparatus (refer to Patent Document 1).

US 2008/015487 discloses a blood purification apparatus with regional citrate anticoagulation comprising a combined calcium, magnesium and citrate sensor in the effluent line of the blood purifier. The composition of the ultrafiltrate is determined on the basis of values measured by this combined sensor. From the determined composition of the ultrafiltrate, the composition of the patient's plasma is back-calculated the citric acid solution supply means and a waste liquid flow rate in the waste liquid circuit, and to determine a supply flow rate of the calcium solution in the calcium solution supply means from the total calcium flow rate of the waste liquid.

US 9,039,647 discloses a blood purification apparatus wherein the supply flow rate (V) of the calcium solution is controlled such that the total amount of calcium of blood returned to the patient (TCAout) is the same as the total amount of calcium of blood entering the blood circuit (TCA). The calculation of the supply flow rate (V) of the calcium solution takes into account the amount of calcium loss from the blood circuit through the blood purifier filter membrane. The calculation of V is dependent both on the measured amount of calcium concentration (B) in the patient's blood plasma, the sieving coefficient (T) of calcium through the filter membrane of the blood purifier and the ultrafiltration flow rate.

Citation List

Patent Document

Patent Document 1: Patent Publication US-A-2016/0121035 (Specification)

Summary

The inventors have devised a simple and accurate determination of a flow rate of calcium supplied to a patient's blood in a blood circuit that, in the above blood purification apparatus.

The present invention has been made in view of the above circumstances, and an object of the present invention is to provide a blood purification apparatus that can simply and accurately determine a flow rate of calcium that is supplied to a patient's blood.

Solution to Problem

**The** inventors conducted extensive studies and as a result, found that it is possible to achieve the above object by calculating a total calcium flow rate of a waste liquid from a total calcium concentration in a waste liquid in a waste liquid circuit when a citric acid solution is supplied and a waste liquid flow rate in the waste liquid circuit and determining a calcium solution supply flow rate from the total calcium flow rate of the waste liquid, and completed the present invention.

Specifically, the present invention includes the following aspects.

(1) A blood purification apparatus including a blood circuit to which a blood purifier is connected and through which blood flows, and a waste liquid circuit through which a waste liquid separated from blood in the blood purifier flows, wherein the blood purification apparatus includes citric acid solution supply means that supplies a citric acid solution to a side upstream from the blood purifier in the blood circuit and calcium solution supply means that supplies a calcium solution to a side downstream from the blood purifier in the blood circuit, wherein the blood purification apparatus is configured to calculate a total calcium flow rate of a waste liquid from a total calcium concentration in the waste liquid in the waste liquid circuit when a citric acid solution is supplied from the citric acid solution supply means and a waste liquid flow rate in the waste liquid circuit, and to determine a calcium solution supply flow rate in the calcium solution supply means from the total calcium flow rate in the waste liquid.

(2) The blood purification apparatus according to (1),
wherein a calcium solution supply flow rate Q in the calcium solution supply means is determined according to a formula: $Q = k*[TCa]on*Qeff$ using a total calcium concentration $[TCa]on$ in the waste liquid in the waste liquid circuit when a citric acid solution is supplied from the citric acid solution supply means, a waste liquid flow rate $Qeff$ in the waste liquid circuit, and a coefficient k.

(3) The blood purification apparatus according to (2),
wherein the coefficient k is determined according to a value obtained by dividing a calcium ion concentration $[iCa]$ off in the waste liquid in the waste liquid circuit when supply of a citric acid solution from the citric acid solution supply means is stopped by a sieving coefficient Sc in the blood purifier.

(4) The blood purification apparatus according to (2), wherein the coefficient k is determined according to a calcium ion concentration in a patient's blood.

(5) The blood purification apparatus according to (4), wherein the coefficient k decreases as the calcium ion concentration in the patient's blood increases.

(6) The blood purification apparatus according to any one of (2) to (5), further including input means that inputs at least a total calcium concentration $[TCa]on$ in the waste liquid in the waste liquid circuit when a citric acid solution is supplied from the citric acid solution supply means and a waste liquid flow rate $Qeff$ in the waste liquid circuit.

(7) The blood purification apparatus according to any one of (1) to (6), wherein an amount of citric acid supplied from the citric acid solution supply means is adjusted using a value obtained by dividing a calcium ion concentration $[iCa]on$ in the waste liquid in the waste liquid circuit when a citric acid solution is supplied from the citric acid solution supply means by a sieving coefficient Sc in the blood purifier.

(8) The blood purification apparatus according to any one of (1) to (7), wherein the condition of the patient's blood is determined using a value D/A, as an index, obtained by dividing a total calcium concentration D in the patient's blood by a value A obtained by dividing a calcium ion concentration $[iCa]off$ in the waste liquid in the waste liquid circuit when supply of a citric acid solution from the citric acid solution supply means is stopped by a sieving coefficient Sc in the blood purifier.

Here, "total calcium" includes all forms of calcium including calcium ions, protein-bound calcium, and ion-bound calcium.

Advantageous Effects of Invention

According to the present invention, it is possible to simply and accurately determine a flow rate of calcium that is supplied to a patient's blood.

**Brief Description of Drawings**

Fig. 1 is an explanatory diagram showing an outline of a configuration of a blood purification apparatus.
Fig. 2 is a block diagram showing an example of a configuration of a control device.
Fig. 3 is a block diagram showing an example of a configuration of a control device including determination means.
Fig. 4 is an explanatory diagram showing an outline of a configuration of another blood purification apparatus.

**Description of Embodiments**

Preferable embodiments of the present invention will be described below with reference to the drawings. Here, the same components are denoted with the same reference numerals, and redundant descriptions will be omitted. In addition, positional relationships such as above, below, left, and right are based on the positional relationships shown in the drawings unless otherwise specified. In addition, dimensional ratios in the drawings are not limited to the illustrated ratios. In addition, the following embodiments are only examples for describing the present invention, and the present invention is not limited to the embodiments, but defined by the technical features as mentioned in claim 1.

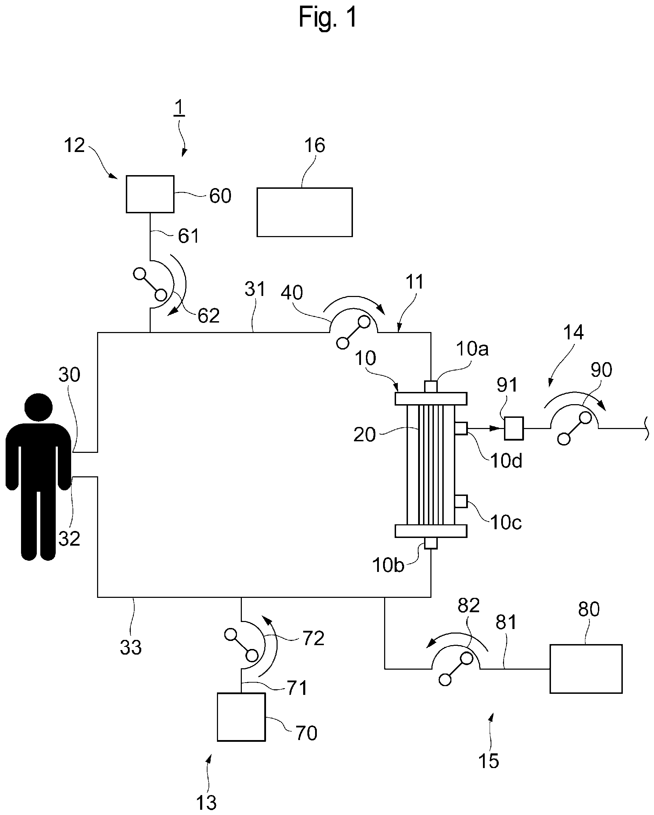

Fig. 1 is an explanatory diagram showing an outline of a configuration of a blood purification apparatus 1 according to the present embodiment. The blood purification apparatus 1 according to the present embodiment is used to perform, for example, hemofiltration.

The blood purification apparatus 1 includes, for example, a blood purifier 10, a blood circuit 11, citric acid solution supply means 12, calcium solution supply means 13, a waste liquid circuit 14, a replacement fluid circuit 15, a control device 16, and the like.

The blood purifier 10 has a blood purification membrane 20 made of a hollow fiber membrane in a cylindrical module. The blood purifier 10 has liquid flow ports 10a and 10b communicating with the primary side (blood side) of the blood purification membrane 20 and liquid flow ports 10c and 10d communicating with the secondary side (waste liquid side) of the blood purification membrane 20.

The blood circuit 11 includes, for example, a blood collection circuit 31 that connects a blood collection unit 30 to the liquid flow port 10a of the blood purifier 10, and a blood return circuit 33 that connects the liquid flow port 10b of the blood purifier 10 to a blood return unit 32. The blood circuit 11 is composed of soft tubes.

For example, a blood pump 40 is provided in the blood collection circuit 31. In the blood pump 40, for example, a tube pump is used.

For example, the citric acid solution supply means 12 includes a solution storage unit 60 in which a citric acid solution is stored, a connection line 61 that connects the solution storage unit 60 to the blood collection circuit 31, and a pump 62 configured to supply a citric acid solution in the solution storage unit 60 to the blood collection circuit 31.

For example, the calcium solution supply means 13 includes a solution storage unit 70 in which a calcium solution is stored, a connection line 71 that connects the solution storage unit 70 to the blood return circuit 33, and a pump 72 configured to supply a calcium solution in the solution storage unit 70 to the blood return circuit 33.

For example, the replacement fluid circuit 15 includes a replacement fluid supply source 80, a replacement fluid line 81 that connects the replacement fluid supply source 80 to the blood return circuit 33, and a replacement fluid pump 82 configured to supply a replacement fluid in the replacement fluid supply source 80 to the blood return circuit 33.

For example, the waste liquid circuit 14 communicates with a waste liquid portion outside the device from the liquid flow port 10d of the blood purifier 10. A waste liquid pump 90 is provided in the waste liquid circuit 14. In addition, in the waste liquid circuit 14, a concentration sensor 91 configured to measure a total calcium concentration and a calcium ion concentration of a waste liquid is provided. The measurement result from the concentration sensor 91 is output to the control device 16. Here, the liquid flow port 10c is closed.

For example, the control device 16 is a microcomputer including a CPU and a memory. For example, the control device 16 can perform a blood purification process by controlling operations of respective devices such as the blood pump 40, the waste liquid pump 90, the concentration sensor 91, the replacement fluid pump 82, the citric acid solution supply means 12, and the calcium solution supply means 13. For example, the control device 16 can perform a blood purification process by executing a program stored in a memory in advance.

For example, the control device 16 determines a calcium solution supply flow rate Q (mmol/h) in the calcium solution supply means 13 using a total calcium concentration $[TCa]on$ (mmol/L) of a waste liquid in the waste liquid circuit 14 when a citric acid solution is supplied from the citric acid solution supply means 12, a waste liquid flow rate Qeff (L/h) in the waste liquid circuit 14, and a coefficient k according to the following Formula (1).

$$Q = k*[TCa]on*Qeff \ldots (1)$$

For example, as shown in Table 1, the coefficient k is determined according to a value A ($[iCa]off/Sc$) obtained by dividing a calcium ion concentration $[iCa]off$ (mmol/L) of a waste liquid in the waste liquid circuit 14 when supply of a citric acid solution from the citric acid solution supply means 12 is stopped by a sieving coefficient Sc of the blood purifier 10.

[Table 1]

| A ($[iCa]off/Sc$) | k |
|---|---|
| A < 0.8 | Abnormal value |
| $0.8 \leq A < 0.9$ | 1.3 |
| $0.9 \leq A < 1.0$ | 1.1 |
| $1.0 \leq A < 1.1$ | 1 |

(continued)

| A ([iCa]off/Sc) | k |
|---|---|
| 1.1 ≤ A < 1.2 | 0.9 |
| 1.2 ≤ A < 1.3 | 0.8 |
| 1.3 ≤ A < 1.4 | 0.7 |

Since a calcium ion concentration [iCa]off of a waste liquid when supply of a citric acid solution is stopped is not influenced by citric acid, a calcium ion concentration in a patient's blood is more accurately estimated. Therefore, in Table 1, when a calcium ion concentration in a patient is relatively low, the coefficient k is larger in order to replenish a larger amount of calcium, and when a calcium ion concentration in a patient is relatively high, the coefficient k is smaller in order replenish a smaller amount of calcium.

Regarding the total calcium concentration [TCa]on and the calcium ion concentration [iCa]off of the waste liquid, those detected by the concentration sensor 91 are used. That is, while a patient's blood is caused to flow through the blood circuit 11 by the blood pump 40, a total calcium concentration [TCa]on of the waste liquid in the waste liquid circuit 14 when a citric acid solution is supplied to the blood circuit 11 from the citric acid solution supply means 12 is detected by the concentration sensor 91, and a detection value of the total calcium concentration [TCa]on is output to the control device 16. In addition, while a patient's blood is caused to flow through the blood circuit 11 by the blood pump 40, a calcium ion concentration [iCa]off of the waste liquid in the waste liquid circuit 14 when supply of a citric acid solution from the citric acid solution supply means 12 is stopped is detected by the concentration sensor 91, and a detection value of the calcium ion concentration [iCa]off is output to the control device 16. Here, regarding the total calcium concentration [TCa]on and the calcium ion concentration [iCa]off of a waste liquid, a waste liquid is sampled and a concentration of the waste liquid may be measured without using the concentration sensor 91.

Regarding the waste liquid flow rate Qeff, a flow rate of the waste liquid pump 90 set in the control device 16 is used. Regarding the waste liquid flow rate Qeff, a rate measured using a flowmeter provided in the waste liquid circuit 14 or a rate obtained by calculation may be used.

For example, the sieving coefficient Sc of the blood purifier 10 may be obtained according to specifications of the blood purifier 10 and the blood purification membrane 20, experiments, calculations, or the like.

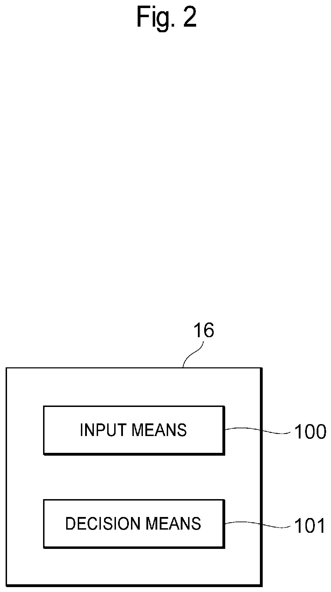

Here, as shown in Fig. 2, the control device 16 includes input means 100 such as a keyboard and a touch panel for inputting a total calcium concentration [TCa]on of a waste liquid in the waste liquid circuit 14 when a citric acid solution is supplied from the citric acid solution supply means 12, a waste liquid flow rate Qeff in the waste liquid circuit 14, a coefficient k, and the like, decision means 101 configured to calculate and determine a calcium solution supply flow rate Q using such input values and Formula (1), and the like.

Next, an example of a blood purification process using the blood purification apparatus 1 will be described.

A patient is punctured with a puncture needle of the blood collection unit 30 and the blood return unit 32, and the blood purification process starts. The blood pump 40 of the blood circuit 11 is activated, a patient's blood is sent to the primary side of the blood purification membrane 20 of the blood purifier 10 through the blood collection circuit 31, and the blood that has passed through the primary side of the blood purification membrane 20 passes through the blood return circuit 33 and returns to the patient. In this case, in the waste liquid circuit 14, the waste liquid pump 90 is activated, and some components of the blood on the primary side of the blood purification membrane 20 pass through the blood purification membrane 20 and are discharged from the blood purifier 10 through the waste liquid circuit 14.

A citric acid solution with a predetermined flow rate is supplied from the citric acid solution supply means 12 to blood flowing from the blood collection unit 30 to the blood purifier 10. Therefore, a calcium ion concentration in the blood is lowered and blood coagulation is prevented. When blood passes through the blood purifier 10, some components of blood containing calcium ions pass through the blood purification membrane 20 and are removed from the blood.

A replacement fluid with a predetermined flow rate is supplied by the replacement fluid circuit 15 to blood flowing from the blood purifier 10 to the blood return unit 32. Thereby, a patient's electrolytes and the like are restored.

In addition, a calcium solution with a predetermined flow rate is supplied from the calcium solution supply means 13 to blood flowing from the blood purifier 10 to the blood return unit 32. Therefore, a concentration of calcium in the blood is restored. Then, the blood is returned from the blood return unit 32 to the patient.

A supply flow rate Q of a calcium solution supplied to a patient's blood is calculated by Formula (1). In order to calculate a supply flow rate Q of a calcium solution, for example, while a patient's blood is caused to flow to the blood circuit 11 by the blood pump 40, supply of a citric acid solution from the citric acid solution supply means 12 is stopped, and a calcium ion concentration [iCa]off in the waste liquid flowing through the waste liquid circuit 14 at this time is detected by the concentration sensor 91. In addition, for example, while a patient's blood is caused to flow through the blood circuit 11 by the blood pump 40, a citric acid solution is supplied from the citric acid solution supply means 12 to the blood circuit 11, and a total calcium concentration [TCa]on in the waste liquid flowing through the waste liquid circuit 14 at this time is detected by the concentration sensor 91. The total calcium concentration [TCa]on and the calcium ion concentration [iCa]off detected by the concentration sensor 91 are output to the control device 16.

Then, in the control device 16, a value A ([iCa]off/Sc) is obtained from the calcium ion concentration [iCa]off and a preset sieving coefficient Sc, and a coefficient k in Table 1 corresponding thereto is obtained. Then, a supply flow rate Q of the calcium solution is obtained according to Formula (1) using the coefficient k, the total calcium concentration [TCa]on of the waste liquid, and the waste liquid flow rate Qeff which is a flow rate of the waste liquid pump 90. Then, a calcium solution with a calcium solution supply flow rate Q is supplied from the calcium solution supply means 12 to the blood circuit 11.

According to the present embodiment, from the total calcium concentration [TCa]on (mmol/L) of the waste liquid of the waste liquid circuit 14 and a waste liquid flow rate Qeff (L/h) in the waste liquid circuit 14, a total calcium flow rate (a total calcium flow rate removed by the blood purifier 10) in the waste liquid is calculated, the total calcium flow rate in the waste liquid is used for estimating a total calcium flow rate that should be excluded from blood in the blood circuit 11, and the estimated total calcium flow rate is used for determining a supply flow rate Q of a calcium solution to be replenished. According to the present embodiment, since a supply flow rate of a calcium solution is calculated based on an amount of calcium that is actually removed from the blood circuit 11, a calcium flow rate to be supplied to a patient's blood (the blood circuit 11) can be determined simply and accurately.

In addition, the calcium solution supply flow rate Q is determined using a formula: Q = k*[TCa]on*Qeff, and the coefficient k is determined according to a value A obtained by dividing a calcium ion concentration [iCa]off in the waste liquid of the waste liquid circuit 14 when supply of a citric acid solution is stopped by the sieving coefficient Sc. In this case, since a calcium ion concentration in a patient's blood is more accurately estimated, and a supply flow rate Q of a calcium solution reflecting the calcium ion concentration are determined, a calcium concentration in the patient's blood is appropriately adjusted. In addition, in this case, since it is not necessary to sample a patient's blood, it is possible to more easily determine a calcium flow rate to be supplied to the patient's blood (the blood circuit 11). Here, a method of determining a coefficient k is not limited thereto, and other methods in which a patient's blood is not sampled may be used.

In the above embodiment, a coefficient k in Formula (1) may be determined according to a calcium ion concentration in the patient's blood. In this case, a calcium ion concentration is obtained from the patient's blood, and a coefficient k is determined based on the calcium ion concentration. For example, the coefficient k decreases as the calcium ion concentration in the patient's blood increases. In this case also, since a calcium ion concentration in the patient's blood is more accurately estimated, and a supply flow rate Q of a calcium solution reflecting the calcium ion concentration is determined, a calcium concentration in the patient's blood is appropriately adjusted. Here, a method in which a coefficient k corresponding to a value A obtained by dividing a calcium ion concentration [iCa]off in the waste liquid of the waste liquid circuit 14 when supply of the citric acid solution is stopped by a sieving coefficient Sc is determined and no patient's blood is sampled and a method of determining a coefficient k according to a calcium ion concentration in the patient's blood may be used in combination.

In the above embodiment, a citric acid supply flow rate Qc in the citric acid solution supply means 12 may be adjusted using a value C obtained by dividing a calcium ion concentration [iCa]on in the waste liquid of the waste liquid circuit 14 when a citric acid solution is supplied from the citric acid solution supply means 12 by a sieving coefficient Sc of the blood purifier 10.

In this case, for example, the citric acid supply flow rate Qc is adjusted based on an adjustment amount Δq of the citric acid supply flow rate determined according to the value C as shown in Table 2. Specifically, the citric acid supply flow rate Qc is a citric acid supply flow rate Qc' before adjustment + adjustment amount Δq.

[Table 2]

| C ([iCa]on/Sc) | Adjustment amount of citric acid supply flow rate Δq [mmol/L] |
| --- | --- |
| C < 0.2 | -0.1 |
| 0.2 ≤ C < 0.3 | 0 |
| 0.3 ≤ C < 0.35 | 0.1 |
| 0.35 ≤ C | 0.2 |

When a citric acid solution is supplied to the patient's blood in the blood circuit 11, the Ci-Ca conjugate in the patient's blood in the blood circuit 11 increases, an amount of calcium ions decreases, and the calcium ion concentration decreases. The value C is an estimated value of the calcium ion concentration in the patient's blood when a citric acid solution is supplied. When the value C is too small, since the calcium ion concentration in the patient's blood is low, an amount of citric acid supplied which causes reduction in the calcium ion concentration decreases, and the calcium ion concentration is restored. When the value C is too large, since the calcium ion concentration in the patient's blood is high, an amount of citric acid supplied which causes reduction in the calcium ion concentration increases and the calcium ion concentration decreases. Thus, according to such examples, it is possible to appropriately adjust the calcium ion concentration in the patient's blood.

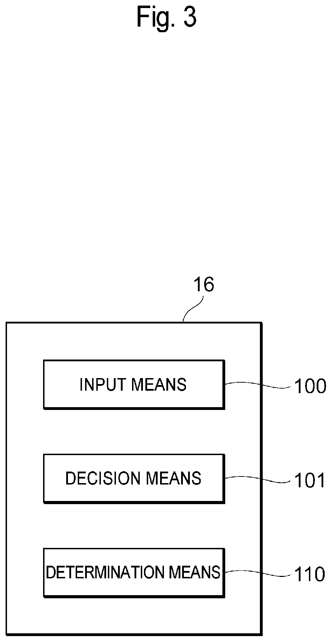

In the above embodiment, the blood purification apparatus 1 may determine the condition of the patient's blood using a value D/A, as an index, obtained by dividing the total calcium concentration D in the patient's blood by the value A obtained by dividing a calcium ion concentration [iCa]off of the waste liquid in the waste liquid circuit 14 when supply of a citric acid solution from the citric acid solution supply means 12 is stopped by the sieving coefficient Sc of the blood purifier 10. In this case, for example, the control device 16 may further include determination means 110 as shown in Fig. 3.

For example, the determination means 110 may determine the condition of the patient's blood according to values of D/A as shown in Table 3.

[Table 3]

| D/A | Condition |
|---|---|
| D/A < 2.2 | Normal value |
| 2.2 ≤ D/A < 2.5 | Alert |
| 2.5 ≤ D/A | Abnormal value |

For example, the patient's blood is not good if a value of (total calcium concentration in blood)/(calcium ion concentration in blood) is too high. That is, when a value of (total calcium concentration in blood)/(calcium ion concentration in blood) is too high, a metabolic disorder of the patient or the like may be caused. The value A obtained by dividing a calcium ion concentration [iCa]off in the waste liquid when supply of a citric acid solution is stopped by the sieving coefficient Sc of the blood purifier 10 can be used as an estimated value of the calcium ion concentration in the patient's blood. Therefore, when the condition of the patient's blood is determined based on a value of D/A, the condition of the patient's blood can be appropriately determined.

While an example in which the present invention is applied to the blood purification apparatus 1 that performs hemofiltration has been described in the above embodiment, the present invention can also be applied to blood purification apparatuses for performing other types of blood processing, for example, a blood purification apparatus for performing hemodialysis and a blood purification apparatus for performing hemodiafiltration.

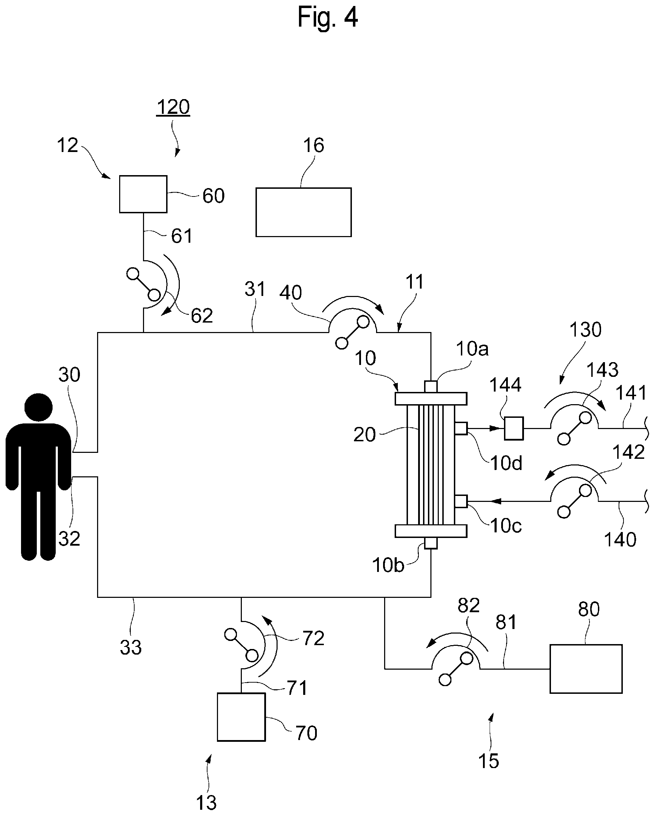

As shown in Fig. 4, a blood purification apparatus 120 for performing hemodiafiltration includes a dialysis fluid circuit 130 that supplies a dialysis fluid to the secondary side of the blood purification membrane 20 of the blood purifier 10 and discharges the dialysis fluid that has passed through the secondary side of the blood purification membrane 20 of the blood purifier 10. The dialysis fluid circuit 130 includes a dialysis fluid supply circuit 140 that is connected to the liquid flow port 10c of the blood purifier 10 and a dialysis fluid discharge circuit 141 connected to the liquid flow port 10d of the blood purifier 10. Pumps 142 and 143 are provided in the dialysis fluid supply circuit 140 and the dialysis fluid discharge circuit 141, respectively. When a dialysis fluid passes through the secondary side of the blood purification membrane 20, predetermined components of the blood that pass through the primary side of the blood purification membrane 20 are discharged to the secondary side of the blood purification membrane 20 and then the blood can be purified. The dialysis fluid discharge circuit 141 in the blood purification apparatus 120 is a waste liquid circuit. A concentration sensor 144 is provided in the dialysis fluid discharge circuit 141. Here, since the other components are the same as those in the above embodiment, they are denoted with the same reference numerals, and descriptions thereof will be omitted.

When a supply flow rate of a calcium solution in the calcium solution supply means 13 is determined, those detected by the concentration sensor 144 of the dialysis fluid discharge circuit 141 are used for a total calcium concentration [TCa]on and a calcium ion concentration [iCa]off of the waste liquid in Formula (1). Here, regarding the total calcium concentration [TCa]on and the calcium ion concentration [iCa]off of the waste liquid, a waste liquid is sampled and a concentration of the waste liquid may be measured without using the concentration sensor 144. Regarding the waste liquid flow rate Qeff, a flow rate of the pump 143 set in the control device 16 is used. Here, for example, the blood purification apparatus for performing hemodialysis has the same configuration as the blood purification apparatus 120 except that it does not include the replacement fluid circuit 15, and it determines a supply flow rate of a calcium solution as in the blood purification apparatus 120, and thus descriptions thereof will be omitted.

Preferable embodiments of the present invention have been described above with reference to the appended drawings.

The circuit configuration of the blood purification apparatus 1 in the above embodiment is not limited to the configuration described in the embodiment. The blood purifier may have a blood purification membrane other than a hollow fiber membrane. The present invention can also be applied to blood purification apparatuses that perform slow continuous hemofiltration, slow continuous hemodialysis, slow continuous hemodiafiltration, slow continuous ultrafiltration (SCUF), or the like.

Industrial Applicability

The present invention is beneficial to providing a blood purification apparatus which can simply and accurately determine a flow rate of calcium that is supplied to a patient.

Reference Signs List

1    Blood purification apparatus
10   Blood purifier
11   Blood circuit
12   Citric acid solution supply means
13   Calcium solution supply means
14   Waste liquid circuit
15   Replacement fluid circuit
16   Control device
20   Blood purification membrane
90   Waste liquid pump
91   Concentration sensor

Claims

1. A blood purification apparatus (1) including a blood circuit (11) to which a blood purifier (10) is connected and through which blood flows, and a waste liquid circuit (14) through which a waste liquid separated from blood in the blood purifier flows, wherein
the blood purification apparatus comprises:

   citric acid solution supply means (12) that supplies a citric acid solution to a side upstream from the blood purifier in the blood circuit; and
   calcium solution supply means (13) that supplies a calcium solution to a side downstream from the blood purifier in the blood circuit,
   **characterized in that** the blood purification apparatus is configured to calculate a total calcium flow rate of a waste liquid from a total calcium concentration of the waste liquid in the waste liquid circuit when a citric acid solution is supplied from the citric acid solution supply means and a waste liquid flow rate in the waste liquid circuit, and to determine a supply flow rate of the calcium solution in the calcium solution supply means from the total calcium flow rate of the waste liquid.

2. The blood purification apparatus according to claim 1,
wherein a calcium solution supply flow rate Q in the calcium solution supply means is determined according to a formula: $Q = k*[TCa]on*Qeff$ using a total calcium concentration $[TCa]on$ in the waste liquid in the waste liquid circuit when a citric acid solution is supplied from the citric acid solution supply means, a waste liquid flow rate $Qeff$ in the waste liquid circuit, and a coefficient k.

3. The blood purification apparatus according to claim 2,
wherein the coefficient k is determined according to a value obtained by dividing a calcium ion concentration $[iCa]$ off in the waste liquid in the waste liquid circuit when supply of a citric acid solution from the citric acid solution supply means is stopped by a sieving coefficient Sc in the blood purifier.

4. The blood purification apparatus according to claim 2,
wherein the coefficient k is determined according to a calcium ion concentration in a patient's blood.

5. The blood purification apparatus according to claim 4,

wherein the coefficient k decreases as the calcium ion concentration in the patient's blood increases.

6. The blood purification apparatus according to any one of claims 2 to 5, further comprising
input means (100) that inputs at least a total calcium concentration [TCa]on in the waste liquid in the waste liquid circuit when a citric acid solution is supplied from the citric acid solution supply means and a waste liquid flow rate Qeff in the waste liquid circuit.

7. The blood purification apparatus according to any one of claims 1 to 6,
wherein an amount of citric acid supplied from the citric acid solution supply means is adjusted using a value obtained by dividing a calcium ion concentration [iCa]on in the waste liquid in the waste liquid circuit when a citric acid solution is supplied from the citric acid solution supply means by a sieving coefficient Sc in the blood purifier.

8. The blood purification apparatus according to any one of claims 1 to 7,
wherein the condition of the patient's blood is determined using a value D/A, as an index, obtained by dividing a total calcium concentration D in the patient's blood by a value A obtained by dividing a calcium ion concentration [iCa]off in the waste liquid in the waste liquid circuit when supply of a citric acid solution from the citric acid solution supply means is stopped by a sieving coefficient Sc in the blood purifier.

**Patentansprüche**

1. Blutreinigungsvorrichtung (1), umfassend einen Blutkreislauf (11), an den ein Blutreiniger (10) angeschlossen ist und durch den Blut fließt, und einen Abfallflüssigkeitskreislauf (14), durch den eine in dem Blutreiniger aus Blut abgetrennte Abfallflüssigkeit fließt, wobei die Blutreinigungsvorrichtung umfasst:

   eine Zitronensäurelösung-Zufuhreinrichtung (12), die eine Zitronensäurelösung zu einer Seite stromaufwärts von dem Blutreiniger in dem Blutkreislauf zuführt; und
   eine Calciumlösung-Zufuhreinrichtung (13), die eine Calciumlösung zu einer Seite stromabwärts von dem Blutreiniger in dem Blutkreislauf zuführt;
   **dadurch gekennzeichnet, dass** die Blutreinigungsvorrichtung so konfiguriert ist, dass sie aus einer Gesamtcalciumkonzentration der Abfallflüssigkeit in dem Abfallflüssigkeitskreislauf eine Gesamtcalciumfließgeschwindigkeit einer Abfallflüssigkeit, wenn eine Zitronensäurelösung aus der Zitronensäurelösung-Zufuhreinrichtung zugeführt wird, und einer Abfallflüssigkeitsfließgeschwindigkeit in dem Abfallflüssigkeitskreislauf berechnen kann und aus der Gesamtcalciumfließgeschwindigkeit der Abfallflüssigkeit eine Zufuhrfließgeschwindigkeit der Calciumlösung in der Calciumlösung-Zufuhreinrichtung bestimmen kann.

2. Blutreinigungsvorrichtung gemäß Anspruch 1, wobei eine Calciumlösung-Zufuhrfließgeschwindigkeit Q in der Calciumlösung-Zufuhreinrichtung gemäß einer Formel bestimmt wird: Q = k*[TCa]on*Qeff unter Verwendung einer Gesamtcalciumkonzentration [TCa]on in der Abfallflüssigkeit in dem Abfallflüssigkeitskreislauf, wenn eine Zitronensäurelösung aus der Zitronensäurelösung-Zufuhreinrichtung zugeführt wird, einer Abfallflüssigkeit-Fließgeschwindigkeit Qeff in dem Abfallflüssigkeitskreislauf und eines Koeffizienten k.

3. Blutreinigungsvorrichtung gemäß Anspruch 2, wobei der Koeffizient k gemäß einem Wert bestimmt wird, der dadurch erhalten wird, dass man eine Calciumionenkonzentration [iCa]off in der Abfallflüssigkeit in dem Abfallflüssigkeitskreislauf, wenn die Zufuhr einer Zitronensäurelösung aus der Zitronensäurelösung-Zufuhreinrichtung unterbrochen ist, durch einen Siebkoeffizienten Sc in dem Blutreiniger dividiert.

4. Blutreinigungsvorrichtung gemäß Anspruch 2, wobei der Koeffizient k gemäß einer Calciumionenkonzentration im Blut eines Patienten bestimmt wird.

5. Blutreinigungsvorrichtung gemäß Anspruch 4, wobei der Koeffizient k mit zunehmender Calciumionenkonzentration im Blut des Patienten abnimmt.

6. Blutreinigungsvorrichtung gemäß einem der Ansprüche 2 bis 5, weiterhin umfassend eine Eingabeeinrichtung (100), die wenigstens eine Gesamtcalciumkonzentration [TCa]on in der Abfallflüssigkeit in dem Abfallflüssigkeitskreislauf, wenn eine Zitronensäurelösung aus der Zitronensäurelösung-Zufuhreinrichtung zugeführt wird, und eine Abfallflüssigkeit-Fließgeschwindigkeit Qeff in dem Abfallflüssigkeitskreislauf eingibt.

**7.** Blutreinigungsvorrichtung gemäß einem der Ansprüche 1 bis 6, wobei die Menge der Zitronensäure, die aus der Zitronensäurelösung-Zufuhreinrichtung zugeführt wird, unter Verwendung eines Werts eingestellt wird, der dadurch erhalten wird, dass man eine Calciumionenkonzentration [iCa]on in der Abfallflüssigkeit in dem Abfallflüssigkeitskreislauf, wenn eine Zitronensäurelösung aus der Zitronensäurelösung-Zufuhreinrichtung zugeführt wird, durch einen Siebkoeffizienten Sc in dem Blutreiniger dividiert.

**8.** Blutreinigungsvorrichtung gemäß einem der Ansprüche 1 bis 7, wobei der Zustand des Bluts des Patienten unter Verwendung eines Werts D/A als Index bestimmt wird, der dadurch erhalten wird, dass man eine Gesamtcalciumkonzentration D in dem Blut des Patienten durch einen Wert A dividiert, der dadurch erhalten wird, dass man eine Calciumionenkonzentration [iCa]off in der Abfallflüssigkeit in dem Abfallflüssigkeitskreislauf, wenn die Zufuhr einer Zitronensäurelösung aus der Zitronensäurelösung-Zufuhreinrichtung unterbrochen ist, durch einen Siebkoeffizienten Sc in dem Blutreiniger dividiert.

**Revendications**

**1.** Appareil de purification du sang (1) incluant un circuit de sang (11) auquel un purificateur de sang (10) est relié et à travers lequel le sang s'écoule, et un circuit de liquide résiduaire (14) à travers lequel s'écoule un liquide résiduaire séparé du sang dans le purificateur de sang, dans lequel l'appareil de purification du sang comprend:

un moyen d'apport de solution d'acide citrique (12) qui apporte une solution d'acide citrique à un côté en amont du purificateur de sang dans le circuit de sang; et
un moyen d'apport de solution de calcium (13) qui apporte une solution de calcium à un côté en aval du purificateur de sang dans le circuit de sang, **caractérisé en ce que**
l'appareil de purification du sang est configuré pour calculer un débit de calcium total d'un liquide résiduaire à partir d'une concentration de calcium total du liquide résiduaire dans le circuit de liquide résiduaire lorsqu'une solution d'acide citrique est apportée par le moyen d'apport de solution d'acide citrique et d'un débit de liquide résiduaire dans le circuit de liquide résiduaire, et pour déterminer un débit d'apport de la solution de calcium dans le moyen d'apport de solution de calcium à partir du débit de calcium total du liquide résiduaire.

**2.** Appareil de purification du sang selon la revendication 1, dans lequel un débit d'apport de solution de calcium Q dans le moyen d'apport de solution de calcium est déterminé selon une formule: $Q = k*[TCa]on*Qeff$ en utilisant une concentration de calcium total [TCa]on dans le liquide résiduaire dans le circuit de liquide résiduaire lorsqu'une solution d'acide citrique est apportée par le moyen d'apport de solution d'acide citrique, un débit de liquide résiduaire Qeff dans le circuit de liquide résiduaire et un coefficient k.

**3.** Appareil de purification du sang selon la revendication 2,
dans lequel le coefficient k est déterminé selon une valeur obtenue en divisant une concentration d'ions calcium [iCa]off dans le liquide résiduaire dans le circuit de liquide résiduaire lorsque l'apport d'une solution d'acide citrique par le moyen d'apport de solution d'acide citrique est arrêté par un coefficient de tamisage Sc dans le purificateur de sang.

**4.** Appareil de purification du sang selon la revendication 2,
dans lequel le coefficient k est déterminé selon une concentration d'ions calcium dans le sang d'un patient.

**5.** Appareil de purification du sang selon la revendication 4,
dans lequel le coefficient k diminue lorsque la concentration d'ions calcium dans le sang du patient augmente.

**6.** Appareil de purification du sang selon l'une quelconque des revendications 2 à 5, comprenant en outre
un moyen d'introduction (100) qui introduit au moins une concentration de calcium total [TCa]on dans le liquide résiduaire dans le circuit de liquide résiduaire lorsqu'une une solution d'acide citrique est apportée par le moyen d'apport de solution d'acide citrique et un débit de liquide résiduaire Qeff dans le circuit de liquide résiduaire.

**7.** Appareil de purification du sang selon l'une quelconque des revendications 1 à 6,
dans lequel une quantité d'acide citrique apportée par le moyen d'apport de solution d'acide citrique est ajustée en utilisant une valeur obtenue en divisant une concentration d'ions calcium [iCa]on dans le liquide résiduaire dans le circuit de liquide résiduaire lorsqu'une solution d'acide citrique est apportée par le moyen d'apport de solution d'acide citrique par un coefficient de tamisage Sc dans le purificateur de sang.

**8.** Appareil de purification du sang selon l'une quelconque des revendications 1 à 7, dans lequel l'état du sang du patient est déterminé en utilisant une valeur D/A, comme indice, obtenue en divisant une concentration de calcium total D dans le sang du patient par une valeur A obtenue en divisant une concentration d'ions calcium [iCa]off dans le liquide résiduaire dans le circuit de liquide résiduaire lorsque l'apport d'une solution d'acide citrique par le moyen d'apport de solution d'acide citrique est arrêté par un coefficient de tamisage Sc dans le purificateur de sang.

# Fig. 1

# Fig. 2

# Fig. 3

16

INPUT MEANS — 100

DECISION MEANS — 101

DETERMINATION MEANS — 110

# Fig. 4

**EP 3 590 563 B1**

**REFERENCES CITED IN THE DESCRIPTION**

**Patent documents cited in the description**

- US 2008015487 A
- US 9039647 B
- US 20160121035 A